# EUROPEAN PATENT SPECIFICATION

(11) **EP 2 367 539 B1**
(45) Date of publication and mention of the grant of the patent: **05.06.2013**
(21) Application number: 09764826.5
(22) Date of filing: 04.12.2009
(51) Int. Cl.: A61K 9/20, A61K 47/10, A61K 31/60

(54) **NOVEL COMPOSITION FOR TREATMENT OF ESSENTIAL THROMBOCYTHEMIA**
NEUE ZUSAMMENSETZUNG ZUR BEHANDLUNG VON ESSENTIELLER THROMBOZYTHÄMIE
NOUVELLE COMPOSITION DESTINÉE À TRAITER LA THROMBOCYTÉMIE ESSENTIELLE

(30) Priority: 05.12.2008 AT 70708 U
(43) Date of publication of application: 28.09.2011
(73) Proprietor: AOP Orphan Pharmaceuticals AG, 1160 Vienna (AT)
(72) Inventor: WIDMANN, Rudolf, A-3002 Purkersdorf (AT)
(74) Representative: Loidl, Manuela Bettina
(86) International application number: PCT/EP2009/066405
(87) International publication number: WO 2010/063824

(56) References cited:
- WO-A1-2008/090569
- WO-A2-2005/004917
- US-A1- 2003 158 261
- US-A1- 2006 246 142

## Description

The present invention relates to a novel pharmaceutical composition comprising anagrelide hydrochloride in combination with lactose monohydrate and its use for the treatment of essential thrombocythemia.

Primary thrombocythemia is a haematopoietic clonal or polyclonal myeloproliferative disease and is diagnosed in 12% of patients with thrombocytosis.

Essential thrombocythemia (ET) is the diagnosis in 45% of patients with primary thrombocytosis (1). In order to clearly differentiate ET from polycythaemia vera (PV) and myelofibrosis (MF), diagnostic criteria were defined by the Polycythaemia vera study group, (2) and later refined by the World Health Organization (WHO) diagnostic guidelines (3, 4).

The WHO diagnostic guidelines refined the PVSG diagnostic critera for ET by the inclusion of bone marrow histopathology and argyrophilic (reticulin and collagen) fibre density to differentiate ET from early pre-fibrotic stages of idiopathic myelofibrosis (IMF) (5, 6, 7).

In patients with secondary thrombocytosis platelet numbers above 1.500.000/µl represent an independent risk factor for thromboembolic and haemorrhagic events (8). Additional independent risk factors are a history of thrombo-embolism or bleeding, microvascular symptoms and age above 65 years (9, 10, 11, 12, 13). Clinical manifestations range from mild symptoms, such as headache, dizziness, and visual disturbances to life threatening complications such as thrombosis, haemorrhage and stroke. The estimated risk for thrombotic episodes is 6.6%/patient year, increases to 15%/patient year in patients over 60 years (14) and is highest in patients with previous history of occlusive episodes (10).

It is generally accepted that high-risk patients should receive platelet-lowering treatment. About 50% of patients show a history of thrombosis or haemorrhage at the time of diagnosis and fall into the high-risk category (15). Considering all independent risk factors such as platelet counts above 1.500.000/µl, age above 65 years and cardiovascular risk factors more than 50% of patients diagnosed with ET require treatment. In addition, symptomatic patients with platelet values below 900.000/µl are at risk for complications and require treatment (16). Large-vessel thrombosis may occur in young patients with platelet counts of less than 900.000/µl (17).

Reduction of platelet counts has been shown to reduce the risk for clinical complications (18). Platelet counts may therefore serve as a surrogate marker for clinical complications. Increasing evidence exists that young patients and patients with platelet counts less than 900.000/µl may benefit from treatment by reducing the risk of clinical complications and progressive vascular disease.

In recent years two treatment options were primarily pursued, hydroxyurea (HU) and Alpha-Interferon. However, hydroxyurea has disadvantages: 1. HU is not selective in lowering platelet numbers, and affects other blood components, 2. an increasing number of reports list severe side effects caused by HU, including leukemia. This is especially troublesome if young patients are treated for long periods of time. 3. Some patients are refractory to the treatment with HU.

Alpha-interferon requires subcutaneous administration, has multiple effects on other cell lineages and is not well tolerated by many individuals (19).

Anagrelide (Imidazo (2,1-b) quinazolin-2 (3H)-one,6,7-dichloro-1,5-dihydromonohydrochloride) is currently registered as a second line treatment option for patients with thrombocythemia. Its mechanism of action is selective for megakaryocytes and platelets. It can be administered orally. No evidence for long-term mutagenicity and leukemogenicity has been reported. Anagrelide has been licensed in the USA since 1997 and Europe since 2001. The currently approved therapeutic indication for Anagrelide in the EU is *"for reduction of elevated platelet counts in at risk essential thrombocythemia patients who are intolerant to their current therapy or whose elevated platelet counts are not reduced to an acceptable level by their current therapy"* (http://www.emea.eu.int/humandocs/Humans/EPAR/xagrid/Xagrid.htm).

Anagrelide was originally developed as an inhibitor of platelet aggregation. Its mode of action involves inhibition of platelet cyclic AMP phosphodiesterase enzyme activity (20). However, this activity does not mediate platelet reduction. The selective platelet reducing activity is restricted to humans, occurs at much lower dose (21) and is mediated through inhibition of maturation of megakaryocytes (22, 23). Anagrelide does not affect megakaryocyte colony formation and platelet survival at therapeutic concentrations (22). The exact mechanism of action is currently unknown. However, metabolites of Anagrelide appear to have strong platelet lowering activity and therefore are likely to contribute to its action (24, 25, 26).

Current evidence and risk-benefit balance in the majority of patients favour Anagrelide as an appropriate treatment choice in a wider patient population. This also includes patients at risk younger than 60 years of age who are reluctant to start treatment with agents that pose a potential leukemogenic risk, such as hydroxyurea.

Anagrelide is extensively metabolized in vivo and more than five metabolites can be identified in urine by HPLC analysis (27). Two of these metabolites were identified, i.e. the biologically active 3-hydroxyanagrelide and the inactive 2-amino-5,6-dichloro-3,4-dihydroquinazolone (also called RL 603)(26).

Recent preclinical and clinical studies of pharmacologic aspects of anagrelide raise important questions regarding its mode of action and safety profile (26, 28, 29, 30, 31). It has been suggested that at least one already identified metabolite, namely 3-hydroxyanagrelide, contributes to the main pharmacological actions in vivo, which are platelet reduction and PDE 3 inhibitory activity, whereas RL 603 lacks these activities (24). Anagrelide is rapidly converted to 3-hydroxyanagrelide which reaches peak plasma levels about 45 min later compared to the parent drug. It is highly likely that both compounds contribute to the pharmacological activity in vivo. Both anagrelide and 3-hydroxyanagrelide are rapidly eliminated from the blood, and there is no evidence for accumulation. It takes days to weeks until optimal platelet control is reached (29, 30). Therefore it is unlikely that platelet reduction is mediated by a direct pharmacologic activity. The molecular target mediating the long lasting platelet reducing effect on megakaryocytes is currently unknown, but may include modulation of c-MPL function resulting in altered thrombopoetin affinity (28). This hypothesis is supported by the finding that it takes 4 to 7 days after discontinuation of anagrelide for platelets to return to pretherapy values.

In contrast, cAMP-dependent PDE 3 has been identified as the molecular target mediating acute effects of anagrelide on the cardiovascular system and on platelet aggregation (20). Headache, dizziness and palpitations represent frequent adverse events occurring in about 15 to 44% of patients during the first weeks of treatment and are probably mediated by mild cardiovascular and cerebral effects of anagrelide at therapeutic dose levels. The improvement of these side effects within 2 to 3 weeks is consistent with the development of tachyphylaxis (29), which also has been observed with other PDE 3 inhibitors (32). However, the rate and severity of cardiovascular side effects like hypotension increases with dose escalation above 5 mg, suggesting a correlation with plasma levels of anagrelide or, more importantly, 3-hydroxyanagrelide (29). Cases of reversible high output heart failure have been reported in patients treated with anagrelide (33, 34). A risk of heart failure after prolonged treatment was also observed in patients treated with milrinone, another positive inotropic PDE 3 inhibitor (35). However, 3-hydroxyanagrelide is considerably more potent than anagrelide as an inhibitor of PDE 3 (26). It may be hypothesized that 3-hydroxyanagrelide primarily mediates the cardiovascular side effects in patients treated with anagrelide. In particular, unpleasant cardiovascular adverse events such as palpitations, dizziness or headache may be caused primarily by 3-hydroxyanagrelide. These are troublesome for patients especially at the initiation of therapy and may be related to a reported discontinuation rate of up to 28% (29, 30).

When using different formulations of anagrelide, differences in adverse events have been reported, and the rate of patients discontinuing anagrelide was reported to range from 8 to 28% (29, 30, 31). Different pharmacokinetic properties can be correlated with pharmacodynamic differences with respect to tolerability and platelet reduction. A delayed absorption rate of anagrelide will be associated with reduced peak and overall plasma levels of anagrelide as well as significantly lower levels of 3-hydroxyanagrelide. As a consequence, a markedly lower rate of PDE 3-dependent acute side effects will be expected to occur.

US 6,287,599 and US 2004/0062800 disclose pharmaceutical compositions with sustained release and reduced pH dependent dissolution profiles. Amongst others anagrelide containing tablets are described wherein 2.44mg/tablet anagrelide HCl is contained. WO2005/112917A1 describes compositions containing selective cytokine inhibitory drugs for the treatment of myeloproliferative diseases wherein anagrelide is present as second active agent.
US2007/104782A1 and WO2007/016350A describe pH independent release tablet formulations with enhanced mechanical properties containing a methacrylic acid copolymer.
US2005/0249814A1 and US2005/0008704A1 disclose formulations comprising a compound with at least one carboxylic acid moiety having rapid dissolution upon contact with physiological solvents.

It is an object of the present invention to make available novel compositions of anagrelide with non-immediate and highly consistent release properties, which can be used for the prevention and treatment of symptoms related to chronic myeloproliferative disorders, such as essential thrombocythemia, but which lack some of the well known side effects of anagrelide treatment attributable to its main metabolite, 3-hydroxyanagrelide.

The object is achieved by the provision of the embodiments of the present application.

The invention relates to novel formulations of anagrelide resulting in a non-immediate release of the active ingredient of anagrelide wherein anagrelide HCl is of small particle size. It has been shown that pharmaceutical compositions comprising anagrelide in combination with lactose monohydrate provide a formulation with reduced release characteristics when administered to a patient.

Optionally, the pharmaceutical composition can further comprise microcristalline cellulose and pyrrolidones like povidone and/or crospovidone.

### Figures:

Figure 1: Plasma anagrelide concentrations after the ingestion of present (P) or novel (N) anagrelide formulations (mean ± SE). Cmax plasma values were significantly different (p<0.001) after administration of present or novel formulation, respectively.
Figure 2: Plasma 3-hydroxyanagrelide (3-OH) concentrations after the ingestion of present (P) or novel (N) anagrelide formulations (mean ± SE). Cmax plasma values were significantly different (p<0.001) after administration of present or novel formulation, respectively.

### Detailed description of the invention

The invention provides a pharmaceutical composition comprising anagrelide hydrochloride particles povidone, crospovidone, microcristalline cellulose, magnesium steocrate wherein at least 90% of said anagrelide particles are smaller than 10 µm in diameter and at least 60mg lactose monohydrate.
Specifically, anagrelide is contained as anagrelide hydrochloride in an amount of less than 1mg, preferably less than 0.70mg, preferably less than 0.65mg, preferably between 0.54 and 0.60mg, preferably in an amount of about 0.57mg. Anagrelide HCl according to the present invention is present in the form of micronized anagrelide having a particle size of less than 15 µm, preferably less than 10 µm. According to a specific embodiment of the invention the mean particle size of the anagrelide in the preparation is about 5 µm.
The use of small particles of less than 15 µm, specifically less than 10 µm of anagrelide is specifically advantageous for pharmaceutical preparations containing low concentrations of anagrelide as thereby a highly homogenous distribution and/or consistent release of the active ingredient anagrelide is provided when administered.
The term "mean particle size of about 5 µm" is defined according to the invention as at least 50% of the particles are smaller than 5 µm and at least 90% are smaller than 10 µm in diameter.

The terms anagrelide, anagrelide particles and anagrelide HCl are terms used for the active compound Imidazo (2,1-b) quinazolin-2 (3H)-one,6,7-dichloro-1,5-dihydromonohydrochloride according to the present invention.

Micronization is known in the art as the process of reducing the average diameter of a solid material's particles.According to the invention "micronization" is performed by known techniques, for example by Rapid Expansion of Supercritical Solutions method, Supercritical Anti-Solvent method and Particles from Gas Saturated Solutions method. Lactose monohydrate can be contained in amount of at least 60mg, preferably of at least 90mg, preferably 93,9mg.
The composition further contains povidone in an amount between 4 and 13mg, preferably in an amount of 5.5 and 6.6mg, preferably 6mg, and crospovidone, in an amount between 3.5 and 13mg, preferably in an amount between 5 and 6mg, preferably in an amount of 5.5mg. The composition also contains microcristalline cellulose. Specifically, the presence of pyrrolidones, more specifically of linked pyrrolidones can contribute to the advantageous non-immediate release properties of the formulation.
The microcristalline cellulose can be contained in an amount between 13 and 39mg, preferably in an amount between 20 and 25mg, preferably 22,5mg.
Further pharmaceutically acceptable substances well known in the art are also be contained in the pharmaceutical composition according to the invention. For example, these substances can be magnesium stearate and anhydrous lactose.

According to a specific embodiment the pharmaceutical composition comprises anagrelide HCl, lactose monohydrate, povidone, crospovidone, microcristalline cellulose and magnesium stearate.

More specifically the pharmaceutical composition comprises
0.57mg anagrelide HCl
93,9mg lactose monohydrate
6.0mg povidone
5.5mg crospovidone
22.5mg microcristalline cellulose
1.5mg magnesium stearate.

It has been shown that this composition shows advantageous characteristics in view of the release properties of the medicament. Anagrelide containing medicaments as presently used are immediate release medicaments wherein more than 90% of anagrelide is released in vitro within the first ten minutes. The novel formulation of anagrelide, in contrast, releases only app. 50% of anagrelide during the first ten minutes in vitro, and takes up to 30 minutes for release of up to approx. 90% in vitro.

Specifically, the pharmaceutical composition according to the invention has a plasma peak release of at least 60 minutes upon oral administration in vivo.

The inventive compostion can be administered orally, in the form of tablets or capsules. According to the present embodiment, capsules like gelatine capsules are a specific pharmaceutical form for oral administration.

It has been shown that this composition shows advantageous characteristics in view of the release properties of the medicament. Anagrelide containing medicaments as presently used are immediate release medicaments wherein anagrelide can be measured in the plasma of the patient within a few minutes after oral administration. Specifically in view of better tolerability and reduction of negative side effects as well as the needs of long term treatment it is preferred to have a medicament that is of non-immediate release resulting in a delayed but more constant supply with anagrelide. Specifically, when using the inventive pharmaceutical composition, anagrelide can be measured in the plasma as plasma peak approximately after 60 minutes, preferably after 90 minutes, more preferably after 120 minutes after the composition is administered to the patient.

Specifically in view of a better tolerability and reduction of negative side effects attributable to 3-hydroxyanagrelide it is preferred to have a medicament that is of non-immediate release resulting in a delayed supply with anagrelide and hence lower peak plasma levels of 3-hydroxyanagrelide.

The anagrelide HCl containing composition according to the invention further shows improved tolerability due to the fact that the concentration of anagrelide HCl is low. The content of anagrelide is less than 1mg, preferably less than 0.70mg, preferably less than 0.65mg, preferably between 0.54 and 0.60mg, preferably in an amount of 0.57mg

Due to the novel release properties of the inventive compositions it is specifically useful for the first line treatment of essential thrombocythemia but can be used for second line treatment as well. The inventive composition is preferred specifically in the treatment of newly diagnosed patients, since unpleasant cardiovascular adverse events such as palpitations, dizziness or headache usually occur during the first weeks of treatment and may lead to early discontinuation of anagrelide therapy or decreased patient compliance.

Alternatively, the inventive composition can also be administered to patients who are intolerant to their current therapy or whose elevated platelet counts are not reduced to an acceptable level by their current therapy.

The recommended starting dose is 1 mg/d for 1 week, and the dose is weekly adjusted until optimal platelet control is achieved. Usually a reduction of platelet counts is achieved at a dose of 1 to 3 mg/d.

The anagrelide HCl containing composition can be administered several times throughout the day until the dose to get reduction of platelet counts is achieved, specifically it is administered in the morning and in the evening.

The examples described herein are illustrative of the present invention and are not intended to be limitations thereon. Different embodiments of the present invention have been described according to the present invention. Many modifications and variations may be made to the techniques described and illustrated herein without departing from the spirit and scope of the invention. Accordingly, it should be understood that the examples are illustrative only and are not limiting upon the scope of the invention.

### Examples:

### In vitro dissolution study:

The analysis was performed according to the European Pharmacopoeia (method 2.9.3, (36)) "dissulotion test for solid dosage forms" via the paddle method, which requires that the solid dosage form is placed into the apparatus containing an appropriate medium (500 ml) at 37+/-0,5°C and a defined operating rate for the paddle. To test the dissolution profiles of present and novel formulation, respectively, for each experiment one 0.5mg capsule was placed into the apparatus containing a medium (0,1 M HCl, containing 0,1% SDS to improve the solubility of anagrelide). Samples were taken at 0, 5, 10, 15, 30 and 60 minutes from 6 experiments per formulation and analyzed by HPLC. The results are presented as %+/-SD of anagrelide released from the nominal amount contained in the capsule and the dissolution profiles were analysed by Student's t-test for paired comparisons.

### Pharmacokinetic study:

A pharmacokinetic study was performed to compare the bioavailability of present and novel formulation of anagrelide in a two period single dose, double-blind cross over study (wash out period 7 days) at a dose of 2 mg (4x0,5mg orally given as one dose). Volunteers were 24 healthy males and females fasted with inclusion and exclusion criteria as recommended by respective guidelines (37). A clinical and laboratory screening was conducted, vital signs taken and a physical examination performed. The estimated sample size of at least 24 subjects gave a power of >0,8 at a CV <0.235 for the bioequivalence interval of 80-120%. A randomisation code was generated by the biostatistician according to which the volunteers were assigned to treatment novel and present formulation, in random order. Treatment compliance was assured by quality assured procedures, Time points for collection of blood were before dosing (at 0 hr), and 20 min, 40 min, 1 hr, 1 hr 20, 1 hr 40, 2 hr, 2 hr 30, 3 hr, 3 hr 30, 4 hr, 5 hr, 6 hr, 8 hr, 12 hr and 24 hr after administration of the drug. Blood samples were immediately centrifuged for 10 min at 3000rpm at +4oC. Adverse events were recorded by clinical research personal blinded to treatment. The statistical plan defined that all primary parameters i.e. the pharmacokinetic parameters were to be presented with a 90%CI, and for all additional parameters descriptive statistics was used. Pharmacokinetic parameters (AUC0-∞, Cmax, tmax, and t1/2) were calculated with Kinetica 2000® Version 4.2 (Innaphase Clinical Information Engineering, Philadelphia, PA, USA). Statistical analysis for bioequivalence of the two formulations was performed by Analyses of Variance (ANOVA) on values of target parameters in order to estimate residual error and thus to construct confidence intervals including evaluation of the presence of period or sequence effects. The following programs were used: for the statistical analysis SAS® Version 8.1 (SAS® Institute, Cary, NC, USA), for the test of normality distribution of calculated pharmacokinetic parameter data SAS® PROC INSIGHT (Distribution Test), for ANOVA calculations of the pharmacokinetic parameter data SAS® PROC GLM (General Linear Models) or PROC MIXED (Mixed Models) and for the non-parametric analyses of tmax the EQUIV-Test® (Statistical Solutions, Broadway, MA, USA). Bioequivalence was assessed according to current guidelines with predetermined limits of 80 to 125% for the CIs of Test/Reference dugs for Cmax and AUC0-∞ at a power of 80% (37). The number of total adverse events was analysed by a paired Student's T test. (Table 1).

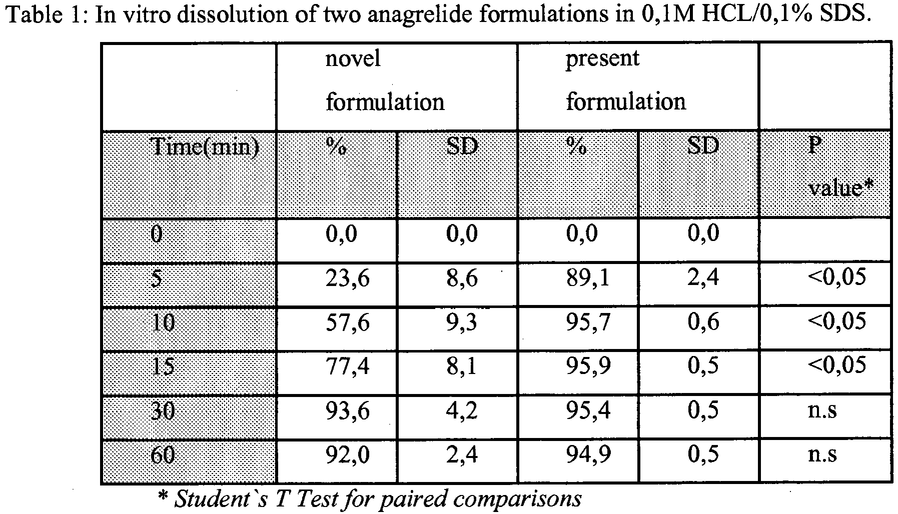

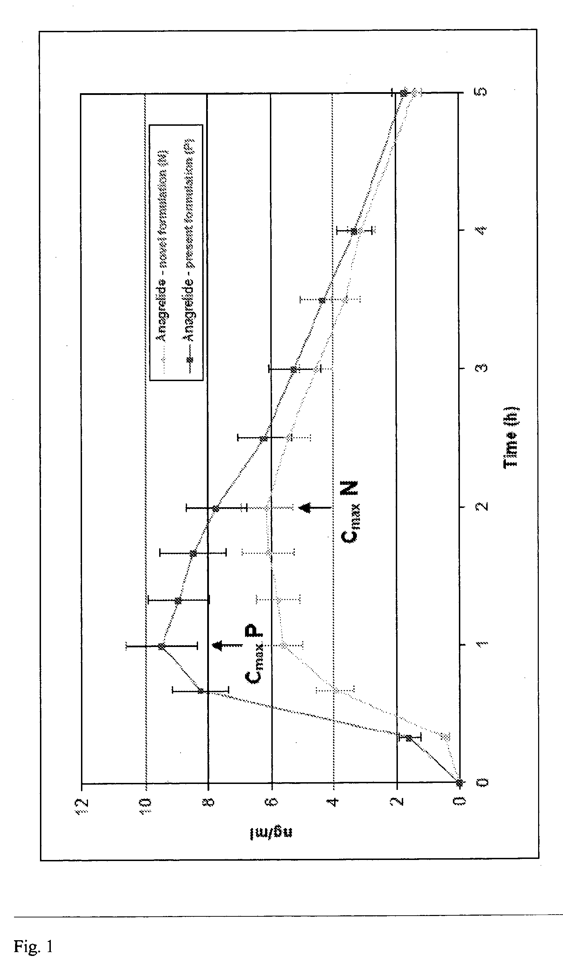

When anagrelide plasma levels were compared in 24 volunteers after the ingestion of novel or present anagrelide formulations, different profiles for both drug formulations became evident: maximum plasma concentration (Cmax) and area-under-the curve (AUCO-∞) were significantly lower (p < 0,001) for the novel formulation when compared to the present formulation (point estimator (PE) 66%, 90% confidence intervals (CI) 58-76% and PE 77%, 90% CI 69-87%, respectively), thus being far outside of the interval required by European guidelines for bioequivalence (80 to 125%) (12) (Figure 1).

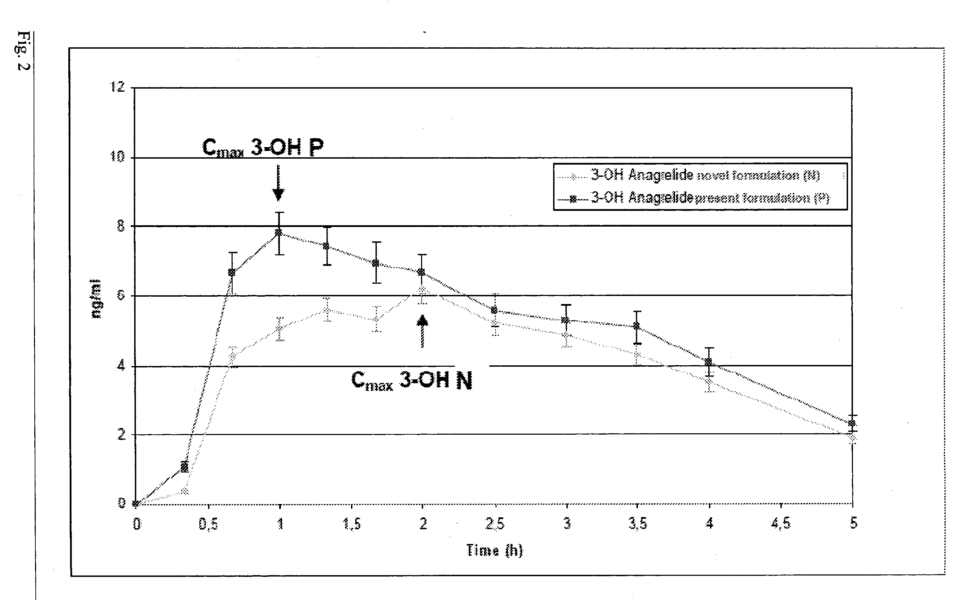

The pharmacokinetic profiles of 3-hydroxyanagrelide after ingestion of the novel formulation were also lower for Cmax (PE 80%; 90% CI 67-84%, p<0,001) and lower AUCO-∞ values (PE 82%; 90% CI 75-87%, p<0,001) compared to present formulation. Peak levels of 3-hydroxyanagrelide following present formulation were reached at one hour (Figure 2). In contrast, following ingestion of novel formulation a delay of 60 minutes was observed.

A total of 75 adverse events were reported in the whole study; 46 occurred in the present formualtion group versus 29 in the novel formulation group (p=0,05, Student's t-Test). The difference in events was most prominent for headache (19 vs.9) and dizziness (12 vs.7) (Table 2).

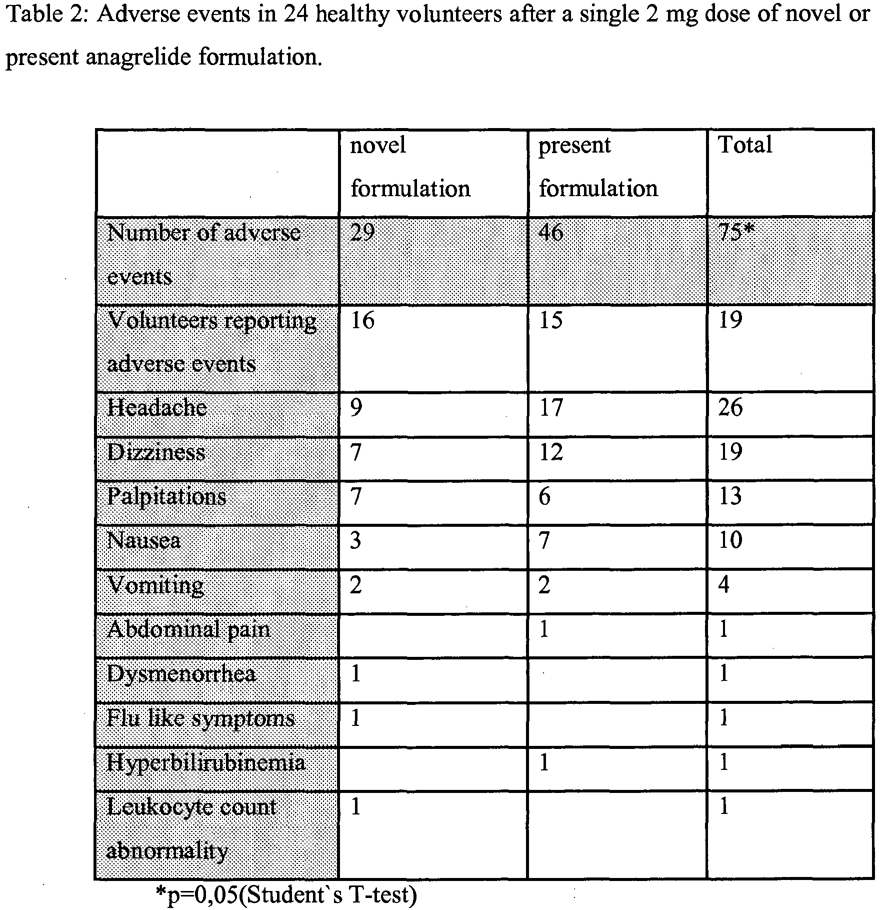

### REFERENCES:

1. GRIESSHAMMER, M., BANGERTER, M., SAUER, T., WENNAUER, R., BERGMANN, L., HEIMPEL, H. (1999). Aetiology and clinical significance of thrombocytosis: analysis of 732 patients with an elevated platelet count. Journal of Internal Medicine 245, 295-300
2. MURPHY, S., PETERSON, P., ILAND, H., LASZLO, J. (1997). Experience of the Polycythemia Vera Study Group with essential thrombocythemia: a final report on diagnostic criteria, survival, and leukemic transition by treatment. Semin Hematol 34, 29-39.
3. IMBERT, M., PIERRE, R., THIELE, J., VARDIMAN, J. W., BRUNNING, R. D., FLANDRIN, G. (2001). Essential thrombocythemia (Lyon: IARC Press).
4. TEFFERI A, THIELE J, ORAZI A, KVASNICKA HM, BARBUI et al. Proposals and rationale for revision of the World Health Organization diagnostic criteria for polycythemia vera, essential thrombocythemia, and primary myelofibrosis. Recommendations from an ad hoc international panel. Blood. 2007; 110:1092-1097.
5. THIELE, J., AND KVASNICKA, H. M. (2003). Chronic myeloproliferative disorders with thrombocythemia: a comparative study of two classification systems (PVSG, WHO) on 839 patients. Ann Hematol 82, 148-152. Epub 2003 Feb 2022.
6. THIELE, J., KVASNICKA, H. M., FISCHER, R. (1999). Histochemistry and morphometry on bone marrow biopsies in chronic myeloproliferative disorders - aids to diagnosis and classification. Ann Hematol 78, 495-506.
7. THIELE, J., KVASNICKA, H. M., ZANKOVICH, R., DIEHL, V. (2000). Relevance of bone marrow features in the differential diagnosis between essential thrombocythemia and early stage idiopathic myelofibrosis. Haematologica 85, 1126-1134.
8. SCHAFER, A. I. (2004). Thrombocytosis. N Engl J Med 350, 1211-1219.
9. BARBUI, T., BAROSI, G., GROSSI, A., GUGLIOTTA, L., LIBERATO, L. N., MARCHETTI, M., MAZZUCCONI, M. G., RODEGHIERO, F., TURA, S. (2004). Practice guidelines for the therapy of essential thrombocythemia. A statement from the Italian Society of Hematology, the Italian Society of Experimental Hematology and the Italian Group for Bone Marrow Transplantation. Haematologica 89, 215-232.
10. BARBUI, T., FINAZZI, G., DUPUY, E., KILADJIAN, J. J., AND BRIERE, J. (1996). Treatment strategies in essential thrombocythemia. A critical appraisal of various experiences in different centers. Leuk Lymphoma 22 Suppl 1:149-60*.*
11. CORTELAZZO, S., FINAZZI, G., RUGGERI, M., VESTRI, O., GALLI, M., RODEGHIERO, F., BARBUI, T. (1995). Hydroxyurea for Patients with Essential Thrombocythemia and a High Risk of Thrombosis. N Engl J Med 332, 1132.
12. CORTELAZZO, S., VIERO, P., FINAZZI, G., D'EMILIO, A., RODEGHIERO, F., BARBUI, T. (1990). Incidence and risk factors for thrombotic complications in a historical cohort of 100 patients with essential thrombocythemia. J Clin Oncol 8, 556.
13. HARRISON , C. N. (2002). Current trends in essential thrombocythaemia. British Journal of Haematology 117, 796-808.
14. LENGFELDER, E., HOCHHAUS, A., KRONAWITTER, U., HOCHE, D., QUEISSER, W., JAHN-EDER, M., BURKHARDT, R., REITER, A., ANSARI, H., HEHLMANN, R. (1998). Should a platelet limit of 600x10(9)/1 be used as a diagnostic criterion in essential thrombocythaemia? An analysis of the natural course including early stages. British Journal of Haematology 100, 15-23.
15. HARRISON, C. N., GALE, R. E., MACHIN, S. J., LINCH, D. C. (1999). A Large Proportion of Patients With a Diagnosis of Essential Thrombocythemia Do Not Have a Clonal Disorder and May Be at Lower Risk of Thrombotic Complications. Blood 93, 417.
16. MILLARD, F. E., HUNTER, C. S., ANDERSON, M., EDELMAN, M. J., KOSTY, M. P., LUIKEN, G. A., MARINO, G. G. (1990). Clinical manifestations of essential thrombocythemia in young adults. Am J Hematol 33, 27-31.
17. JOHNSON, M., GERNSHEIMER, T., JOHANSEN, K. (1995). Essential thrombocytosis: underemphasized cause of large-vessel thrombosis. J Vasc Surg 22, 443-447; discussion 448-449.
18. LAHUERTA-PALACIOS, J. J., BORNSTEIN, R., FERNANDEZ-DEBORA, F. J., GUTIERREZ-RIVAS, E., ORTIZ, M. C., LARREGLA, S., CALANDRE, L., MONTERO-CASTILLO, J. (1988). Controlled and uncontrolled thrombocytosis. Its clinical role in essential thrombocythemia. Cancer 61, 1207-1212.
19. LENGFELDER, E., GRIESSHAMMER, M., HEHLMANN, R. (1996). Interferon-alpha in the treatment of essential thrombocythemia. Leuk Lymphoma 22 Suppl 1:135-42*.*
20. GILLESPIE, E. (1988). Anagrelide: a potent and selective inhibitor of platelet cyclic AMP phosphodiesterase enzyme activity. Biochem Pharmacol 3 7, 2866-2868.
21. SPENCER, C. M., BROGDEN, R. N. (1994). Anagrelide. A review of its pharmacodynamic and pharmacokinetic properties, and therapeutic potential in the treatment of thrombocythaemia. Drugs 47, 809-822.
22. MAZUR, E., ROSMARIN, A., SOHL, P., NEWTON, J., NARENDRAN, A. (1992). Analysis of the mechanism of anagrelide-induced thrombocytopenia in humans. Blood 79, 1931.
23. TEFFERI, A., SILVERSTEIN, M. N., PETITT, R. M., MESA, R. A., SOLBERG, L. A. J. (1997). Anagrelide as a new platelet-lowering agent in essential thrombocythemia: mechanism of actin, efficacy, toxicity, current indications. Semin Thromb Hemost 23, 379-383.
24. ERUSALIMSKY, J. D., HONG, Y., FRANKLIN, R. (2002). Is the platelet lowering activity of anagrelide mediated by its major metabolite 2-amino-5,6-dichloro-3,4-dihydroquinazoline (RL603)? Exp Hematol 30, 625-626; author reply 626-627.
25. LANE, W. J., HATTORI, K., DIAS, S., PEERSCHKE, E. I., MOORE, M. A., BLANSET, D. L., LANG, P. C., PETRONE, M., RAFII, S. (2001). Anagrelide metabolite induces thrombocytopenia in mice by inhibiting megakaryocyte maturation without inducing platelet aggregation. Exp Hematol 29, 1417-1424.
26. WANG, G., FRANKLIN, R., HONG, Y., ERUSALIMSKY, J. D. (2005). Comparison of the biological activities of anagrelide and its major metabolites in haematopoietic cell cultures. Br J Pharmacol.
27. GAVER RC, DEEB G, PITTMANi KA, SMYTH RD. Disposition of anagrelide, an inhibitor of platelet aggregation. Clin Pharmacol Ther. 1981; 29:381 - 6
28. McCARTY JM, MELONE PD, SIMANIS JP, KANAMORI D, DESSYPRIS EN, WARSHAMANA, GREENE GS. A preliminary investigation into the action of anagrelide: thrombopoietin-c-Mpl receptor interactions. Exp Hematol. 2006 34:87-96
29. STEURER M, GASTL G, JEDRZEJCZAK WW, PYTLIK R, LIN W, SCHLOGL E et al. Anagrelide for thrombocytosis in myeloproliferative disorders: a prospective study to assess efficacy and adverse event profile. Cancer. 2004; 101:2239 - 46
30. BIRGEGARD G, BJORKHOLM M, KUTTI J, LARFARS G, LOFVENBERG E, MARKEVAN B et al. Adverse effects and benefits of two years of anagrelide treatment for thrombocythemia in chronic myeloproliferative disorders. Haematologica. 2004; 89:520- 7
31. HARRISON CN, CAMPBELL PJ, BUCK G, WHEATLEY K, EAST CL, BAREFORD D et al. United Kingdom Medical Research Council Primary Thrombocythemia 1 Study. Hydroxyurea compared with anagrelide in high-risk essential thrombocythemia. N Engl J Med. 2005; 353:33 - 45
32. BEAVO JA. cGMP inhibition of heart phosphodiesterase: is it clinically relevant? J Clin Invest. 1995; 95:445
33. JAMES CW. Anagrelide-induced cardiomyopathy. Pharmacotherapy. 2000; 20:1224-7
34. ENGEL PJ, JOHNSON H, BAUGHAM RP, RICHARDS AI. High-output heart failure associated with anagrelide therapy for essential thrombocytosis. Ann Intern Med. 2005; 143:311-3
35. PACKER M, CARVER JR, RODEHEFFER RJ, IVANHOE RJ, DiBIANCO R, ZELDIS SM et al. Effect of oral milrinone on mortality in severe chronic heart failure. The PROMISE Study Research Group. N Engl J Med. 1991; 325: 1468-75
36. European Pharmacopea, 6th edition, 2008, Chapter 2.9.3, 267, Apparatus 2
37. Note for Guidance for the Investigation of Bioavailability and Bioequivalence (CPMP/EWP/QWP/1401/98 (July 2001), http://www.emea.eu.int/pdfs/human/ewp/140198en.pdf

## Claims

1. A pharmaceutical composition which is in the form of a tablet or a capsule comprising anagrelide particles, povidone, crospovidone, microcristalline cellulose, magnesium stearate and at least 60mg lactose monohydrate, wherein at least 90% of said anagrelide particles are smaller than 10µm in diameter.

2. A pharmaceutical composition according to claim 1 comprising anagrelide in an amount of less than 1 mg, preferably less than 0.70mg, preferably less than 0.65mg, preferably between 0.54 and 0.60mg, preferably in an amount of 0.57mg.

3. A pharmaceutical composition according to any one of claims 1 to 2 comprising at least 60mg, preferably at least 90mg, more preferably at least 93,9mg lactose monohydrate.

4. A pharmaceutical composition according to any one of claims 1 to 3 wherein anagrelide has a mean particle size of about 5µm.

5. A pharmaceutical composition according to any one of claims 1 to 4 comprising povidone in an amount of 4 to 13mg, preferably in an amount of 5.5 to 6.6mg, preferably in an amount of 6mg.

6. A pharmaceutical composition according to any one of claims 1 to 5 comprising crospovidone in an amount of 3.5 to 13mg, preferably in an amount between 5 and 6mg, preferably in an amount of 5.5mg.

7. A pharmaceutical composition according to any one of claims 1 to 6 comprising microcristalline cellulose.

8. A pharmaceutical composition according to claim 7 comprising at least 15mg, preferably at least 20mg, preferably at least 22,5mg microcristalline cellulose.

9. A pharmaceutical composition according to any one claims 1 to 8 comprising pharmaceutically acceptable carriers, preferably selected from the group consisting of crospovidone, povidone and magnesium stearate.

10. A pharmaceutical composition according to any one of claims 1 to 9 having a plasma peak release of at least 60 minutes upon oral administration.

11. A pharmaceutical composition comprising 0.57mg anagrelide HCl, 93,9mg lactose monohydrate, 6mg povidone, 5.5mg crospovidone, 22.5mg microscristalline cellulose and 1.5mg magnesium stearate.

12. Use of a pharmaceutical composition according to any one of claims 1 to 11 for the preparation or a medicament for the treatment of essential thrombocythemia.

13. Pharmaceutical composition according to any one of claims 1 to 11 for use in the treatment of essential thrombocythemia.

## Patentansprüche

1. Pharmazeutische Zusammensetzung, die in Form einer Tablette oder einer Kapsel vorliegt, welche Anagrelid-Partikel, Povidon, Crospovidon, mikrokristalline Zellulose, Magnesiumstearat und mindestens 60 mg Laktose-Monohydrat umfasst, wobei mindestens 90 % der Anagrelid-Partikel kleiner als 10 µm im Durchmesser sind.

2. Pharmazeutische Zusammensetzung nach Anspruch 1, welche Anagrelid in einer Menge von weniger als 1 mg, vorzugsweise weniger als 0,70 mg, vorzugsweise weniger als 0,65 mg, vorzugsweise zwischen 0,54 und 0,60 mg, vorzugsweise in einer Menge von 0,57 mg umfasst.

3. Pharmazeutische Zusammensetzung nach einem der Ansprüche 1 oder 2, welche mindestens 60 mg, vorzugsweise mindestens 90 mg, bevorzugter mindestens 93,9 mg Laktose-Monohydrat umfasst.

4. Pharmazeutische Zusammensetzung nach einem der Ansprüche 1 bis 3, wobei Anagrelid eine mittlere Partikelgröße von etwa 5 µm aufweist.

5. Pharmazeutische Zusammensetzung nach einem der Ansprüche 1 bis 4, welche Povidon in einer Menge von 4 bis 13 mg, vorzugsweise in einer Menge von 5,5 bis 6,6 mg, vorzugsweise in einer Menge von 6 mg umfasst.

6. Pharmazeutische Zusammensetzung nach einem der Ansprüche 1 bis 5, welche Crospovidon in einer Menge von 3,5 bis 13 mg, vorzugsweise in einer Menge zwischen 5 und 6 mg, vorzugsweise in einer Menge von 5,5 mg umfasst.

7. Pharmazeutische Zusammensetzung nach einem der Ansprüche 1 bis 6, welche mikrokristalline Zellulose umfasst.

8. Pharmazeutische Zusammensetzung nach Anspruch 7, welche mindestens 15 mg, vorzugsweise mindestens 20 mg, vorzugsweise mindestens 22,5 mg mikrokristalline Zellulose umfasst.

9. Pharmazeutische Zusammensetzung nach einem der Ansprüche 1 bis 8, welche pharmazeutisch akzeptable Trägerstoffe, vorzugsweise ausgewählt aus der Gruppe bestehend aus Crospovidon, Povidon und Magnesiumstearat, umfasst.

10. Pharmazeutische Zusammensetzung nach einem der Ansprüche 1 bis 9, welche bei oraler Verabreichung eine Spitzenplasmafreisetzung von mindestens 60 Minuten aufweist.

11. Pharmazeutische Zusammensetzung, welche 0,57 mg Anagrelid-HCl, 93,9 mg Laktose-Monohydrat, 6 mg Povidon, 5,5 mg Crospovidon, 22,5 mg mikrokristalline Zellulose und 1,5 mg Magnesiumstearat umfasst.

12. Verwendung einer pharmazeutischen Zusammensetzung nach einem der Ansprüche 1 bis 11 für die Herstellung eines Medikamentes zur Behandlung essentieller Thrombozythämie.

13. Pharmazeutische Zusammensetzung nach einem der Ansprüche 1 bis 11 zur Verwendung bei der Behandlung essentieller Thrombozythämie.

## Revendications

1. Composition pharmaceutique qui se trouve sous la forme d'un comprimé ou d'une capsule comprenant des particules d'anagrélide, de la povidone, de la crospovidone, de la cellulose microcristalline, du stéarate de magnésium et au moins 60 mg de lactose monohydraté, dans laquelle au moins 90 % desdites particules d'anagrélide ont un diamètre inférieur à 10 µm.

2. Composition pharmaceutique selon la revendication 1, comprenant de l'anagrélide en une quantité inférieure à 1 mg, de préférence inférieure à 0,70 mg, de préférence inférieure à 0,65 mg, de préférence comprise entre 0,54 et 0,60 mg, de préférence en une quantité de 0,57 mg.

3. Composition pharmaceutique selon l'une quelconque des revendications 1 à 2, comprenant au moins 60 mg, de préférence au moins 90 mg, de manière davantage préférée au moins 93,9 mg de lactose monohydraté.

4. Composition pharmaceutique selon l'une quelconque des revendications 1 à 3, dans laquelle l'anagrélide a une taille moyenne de particules d'environ 5 µm.

5. Composition pharmaceutique selon l'une quelconque des revendications 1 à 4, comprenant de la povidone en une quantité de 4 à 13 mg, de préférence en une quantité de 5,5 à 6,6 mg, de préférence en une quantité de 6 mg.

6. Composition pharmaceutique selon l'une quelconque des revendications 1 à 5, comprenant de la crospovidone en une quantité de 3,5 à 13 mg, de préférence en une quantité comprise entre 5 et 6 mg, de préférence en une quantité de 5,5 mg.

7. Composition pharmaceutique selon l'une quelconque des revendications 1 à 6, comprenant de la cellulose microcristalline.

8. Composition pharmaceutique selon la revendication 7, comprenant au moins 15 mg, de préférence au moins 20 mg, de préférence au moins 22,5 mg de cellulose microcristalline.

9. Composition pharmaceutique selon l'une quelconque des revendications 1 à 8, comprenant des véhicules pharmaceutiquement acceptables, de préférence choisis dans le groupe constitué de la crospovidone, de la povidone et du stéarate de magnésium.

10. Composition pharmaceutique selon l'une quelconque des revendications 1 à 9 ayant un pic de libération dans le plasma d'au moins 60 minutes après administration orale.

11. Composition pharmaceutique comprenant 0,57 mg d'anagrélide HCl, 93,9 mg de lactose monohydraté, 6 mg de povidone, 5,5 mg de crospovidone, 22,5 mg de cellulose microcristalline et 1,5 mg de stéarate de magnésium.

12. Utilisation d'une composition pharmaceutique selon l'une quelconque des revendications 1 à 11, pour la préparation d'un médicament destiné au traitement de la thrombocytémie essentielle.

13. Composition pharmaceutique selon l'une quelconque des revendications 1 à 11, destinée à être utilisée dans le traitement de la thrombocytémie essentielle.
